(19) Europäisches Patentamt
European Patent Office
Office européen des brevets

(11) Veröffentlichungsnummer : **0 051 782**
**B1**

(12) # EUROPÄISCHE PATENTSCHRIFT

(45) Veröffentlichungstag der Patentschrift : **23.05.84**

(51) Int. Cl.³ : **C 07 C 85/24**, C 07 C 87/60, C 07 C 93/14

(21) Anmeldenummer : **81108888.9**

(22) Anmeldetag : **24.10.81**

(54) **Verfahren zur Herstellung von m-halogensubstituierten Anilinen.**

(30) Priorität : **08.11.80 DE 3042242**

(43) Veröffentlichungstag der Anmeldung : **19.05.82 Patentblatt 82/20**

(45) Bekanntmachung des Hinweises auf die Patenterteilung : **23.05.84 Patentblatt 84/21**

(84) Benannte Vertragsstaaten : **FR**

(56) Entgegenhaltungen :
**EP-B- 0 015 219**
**DE-A- 2 441 650**
**DE-A- 2 549 900**
**US-A- 4 193 937**

(73) Patentinhaber : **BAYER AG**
**Konzernverwaltung RP Patentabteilung**
**D-5090 Leverkusen 1 Bayerwerk (DE)**

(72) Erfinder : **Wedemeyer, Karlfried, Dr.**
**Bilharzstrasse 7**
**D-5000 Koeln 80 (DE)**
Erfinder : **Hagedorn, Ferdinand, Dr.**
**Domblick 16**
**D-5090 Leverkusen 31 (DE)**
Erfinder : **Evertz, Werner**
**Wolfstrasse 1**
**D-4019 Monheim (DE)**

**EP 0 051 782 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Herstellung von m-halogensubstituierten Anilinen durch selektive Enthalogenierung von höherhalogenierten Anilinen.

Aus der DE-PS 2 503 145 und der DE-PS 2 503 187 ist es bekannt, m-chlorsubstituierte Analine durch selektive Enthalogenierung von höherhalogenierten Anilinen in saurem oder neutralem Medium in Gegenwart von Edelmetallkatalysatoren und gegebenenfalls in Gegenwart von Schwefel und/oder Schwefelverbindungen herzustellen.

Die in der DE-PS 2 503 145 und der DE-PS 2 503 187 beschriebenen Verfahren arbeiten allerdings nur bei hohen Temperaturen und Drücken, d. h. bei Temperaturen von über 200 °C und Drücken von über 60 bar, wirtschaftlich (vgl. hierzu auch die Beispiele der genannten Patentschriften). Da die dabei angewandten hohen Reaktionstemperaturen und Drücke z. B. die Behältermaterialien besonders stark beanspruchen, wird die technische Durchführung dieser Verfahren erschwert.

Weiterhin ist aus der DE-OS 3 003 960 ein Verfahren zur Herstellung von m-chlorsubstituierten Anilinen bekannt, bei dem die hydrierende Enthalogenierung in Gegenwart einer stark erhöhten Chloridionen-Konzentration durchgeführt wird. Dieses Verfahren hat den Nachteil, daß nur geringe Konzentrationen der zur Enthalogenierung eingesetzten Verbindung in Lösungen mit relativ hoher Chloridionen-Konzentration angewendet werden können was zu schlechten Raum/Zeit-Ausbeuten und zu erhöhter Korrosion der Apparaturen führt. Das einzige Beispiel dieser Anmeldung läßt vermuten, daß nur in Gegenwart von Lithiumchlorid gute Ergebnisse erzielt werden. Bei dieser Verfahrensweise erhält man jedoch große Mengen an nicht-trennbaren Salzgemischen, deren Aufarbeitung und Beseitigung besondere ökologische Probleme aufwirft.

In der europäischen Patentanmeldung 0 015 219 wird ein Verfahren beansprucht, bei dem die katalytisch hydrierende Enthalogenierung in Gegenwart von Jodid- oder Bromidionen durchgeführt wird. Dieses Verfahren hat den Nachteil, daß man zusätzlich zum während der Reaktion entstehenden Chlorwasserstoff noch größere Mengen an Salzsäure zusetzen muß, wodurch einmal ein erhöhter Zwangsanfall an Chlorwasserstoff bzw. daraus durch Neutralisation gebildetem Salz entsteht, zum anderen die Korrosionsgefahr für die verwendeten Apparaturen erhöht wird. Ein weiterer, wesentlicher Nachteil besteht darin, daß nur geringe Konzentrationen der zur Enthalogenierung eingesetzten Verbindung im Reaktionsgemisch angewendet werden können (vgl. Beispiele 1 bis 15 : maximal 3 Gew.-%). Will man jedoch größere Mengen an Ausgangsprodukt enthalogenieren, so ist es erforderlich, eine große Menge an Katalysator, bezogen auf die zu enthalogenierende Verbindung, einzusetzen (vgl. Beispiel 16 ; Konzentration an Tetrachlornitrobenzol im Gemisch : 12 %, Katalysatormenge : 57 Gew.-%, bezogen auf Tretrachlornitrobenzol). Wie den obigen Ausführungen zu entnehmen ist, handelt es sich bei dem in der europäischen Patentanmeldung 0 015 219 beschriebenen Verfahren um ein technisch und wirtschaftlich wenig geeignetes Verfahren.

Es wurde nun ein Verfahren zur Herstellung von metahalogensubstituierten Anilinen durch Umsetzung von Anilinen der Formel (I)

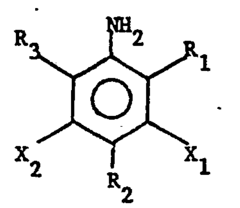

(I)

worin

$X_1$ und $X_2$ gleich oder verschieden sind und für Chlor, Brom, Jod, Wasserstoff oder einen gegebenenfalls substituierten Alkyl-, Aryl-, Aralkyl-, Alkoxy- oder Aralkoxyrest stehen, wobei bei der Herstellung von in einer m-Stellung durch Halogen substituierten Anilinen einer der Reste $X_1$ oder $X_2$ für Chlor, Brom oder Jod steht und bei der Herstellung von 3,5-dihalogensubstituierten Anilinen $X_1$ und $X_2$ für Chlor, Brom oder Jod stehen,

$R_1$, $R_2$ und $R_3$ gleich oder verschieden sind und für Chlor, Brom, Jod, Wasserstoff oder einen gegebenenfalls substituierten Alkyl-, Aryl-, Aralkyl-, Alkoxy- oder Aryloxyrest stehen, wobei wenigstens einer der Reste $R_1$, $R_2$ oder $R_3$ für Chlor, Brom oder Jod steht,

mit Wasserstoff im sauren Medium in Gegenwart von gegebenenfalls auf Trägern aufgebrachten Edelmetallen in elementarer oder gebundener Form und gegebenenfalls in Gegenwart von inerten organischen Lösungs- und/oder Verdünnungsmitteln und/oder Wasser bei erhöhter Temperatur und erhöhten Drücken gefunden, das dadurch gekennzeichnet ist, daß man die Umsetzung in Gegenwart von Jodwasserstoff und/oder Jodwasserstoff liefernden Verbindungen und bei gleichzeitiger Gegenwart von gegebenenfalls substituierten Phenolen durchführt.

Nach dem erfindungsgemäßen Verfahren erhält man m-halogen-substituierte Aniline der Formel (II)

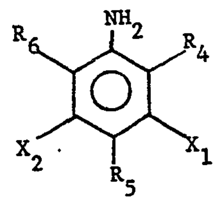

(II)

worin

$X_1$ und $X_2$ die oben angegebene Bedeutung haben, und

$R_4$, $R_5$ und $R_6$ gleich oder verschieden sind und für Wasserstoff oder für einen gegebenenfalls substituierten Alkyl-, Aryl-, Aralkyl-, Alkoxy- oder Aryloxyrest stehen.

Gegebenenfalls substituierte aliphatische Reste ($R_1$, $R_2$, $R_3$, $R_4$, $R_5$ und $R_6$) können geradkettige oder verzweigte aliphatische Reste mit 1 bis 12, bevorzugt mit 1 bis 6 Kohlenstoffatomen, und cycloaliphatische Reste mit 5 bis 8, bevorzugt 5 und 6, Kohlenstoffatomen im Ring sein. Beispielsweise seien der Methyl-, der Ethyl-, der Propyl-, der Isopropyl-, der Butyl-, der Pentyl-, der Hexyl-, der Octyl-, der Nonyl-, der Decyl-, der Dodecyl-, der Cyclopentyl-, der Cyclohexyl-, der Cycloheptyl- und der Cyclooctylrest genannt.

Gegebenenfalls substituierte aromatische Reste ($R_1$, $R_2$, $R_3$, $R_4$, $R_5$ und $R_6$) können Reste aus der Benzolreihe, bevorzugt der Phenyl- oder der Naphtylrest, sein.

Gegebenenfalls substituierte Arylkylreste ($R_1$, $R_2$, $R_3$, $R_4$, $R_5$ und $R_6$) können solche mit 7 bis 18 Kohlenstoffatomen sein, deren aliphatischer Teil 1 bis 6 Kohlenstoffatome, vorzugsweise 1 bis 3 Kohlenstoffatome, enthält und deren aromatischer Teil einen Rest der Benzolreihe, vorzugsweise den Phenyl- oder den Naphthylrest, darstellt. Beispielsweise seien die folgenden Aralkylreste genannt :

der Benzyl-, der m-Ethyl-phenyl-,
der γ-Propyl-phenyl-,
der β-Phenyl-n-hexyl-,
der β-[Naphthyl-(1)]-ethyl-,
der ω-Butylphenyl-,
der ω-Pentyl-phenyl- und
der ω-Hexyl-phenylrest.

Für gegebenenfalls substituierte Alkoxyreste ($R_1$, $R_2$, $R_3$, $R_4$, $R_5$ und $R_6$) können sowohl geradkettige und verzweigte Reste mit 1 bis 12, bevorzugt mit 1 bis 6 Kohlenstoffatomen als auch cycloaliphatische Reste mit 5 und 6 Kohlenstoffatomen, im Ring stehen. Beispielsweise sei der Methoxy-, der Ethoxy-, der Propoxy-, der Isopropoxy-, der Butoxy-, der tert.-Butoxy-, der Pentoxy-, der Hexoxy-, der Oxy-, der Nonoxy-, der Decoxy-, der Dodecoxy-, der Cyclopentoxy- und der Cyclohexoxyrest genannt.

Als gegebenenfalls substituierte Aryloxyreste ($R_1$, $R_2$, $R_3$, $R_4$, $R_5$ und $R_6$) seien Reste aus der Benzolreihe, bevorzugt der Phenoxyrest, genannt.

Als Substituenten der vorstehend aufgeführten Alkyl-, Aryl-, Aralkyl-, Alkoxy- oder Aralkoxyreste kommen beispielsweise die Aminogruppe, die Hydroxylgruppe, geradkettige oder verzweigte Alkylreste mit bis zu 12, bevorzugt bis zu 6 Kohlenstoffatomen, cycloaliphatische Reste, bevorzugt mit 5 und 6 Kohlenstoffatomen im Ring, und Arylreste, bevorzugt der Phenylrest, in Frage.

Bevorzugte Aniline, die in das erfindungsgemäße Verfahren eingesetzt werden können, sind Verbindungen der Formel (III)

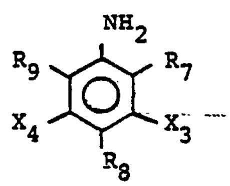

(III)

worin

$X_3$ und $X_4$ gleich oder verschieden sind und für Chlor, Brom, Jod oder Wasserstoff stehen, wobei bei der Herstellung von 3-Halogenanilinen einer der Reste $X_3$ oder $X_4$ für Chlor, Brom oder Jod steht und bei der Herstellung von 3,5-Dihalogenanilinen $X_3$ und $X_4$ für Chlor, Brom oder Jod stehen,

$R_7$, $R_8$ und $R_9$ gleich oder verschieden sind und für Chlor, Wasserstoff, die Methyl- oder die Phenylgruppe oder den Rest

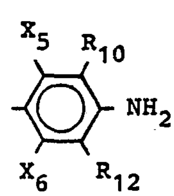

oder

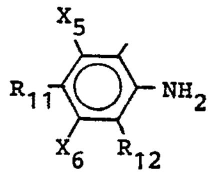

stehen, worin $X_5$ und $X_6$ gleich oder verschieden sind und für Chlor, Brom, Jod oder Wasserstoff stehen, $R_{10}$, $R_{11}$ und $R_{12}$ gleich oder verschieden sind und für Chlor, Wasserstoff, die Methyl- oder die Phenylgruppe stehen, wobei wenigstens einer der Reste $R_7$, $R_8$ oder $R_9$ für Chlor, Brom oder Jod steht.

Die Polyhalogenaniline der Formel (I), die für das erfindungsgemäße Verfahren Verwendung finden können, sind bekannt und leicht zugänglich. Beispielsweise seien genannt :

2,3-Dichlor-anilin,
2,5-Dichlor-anilin,
3,4-Dichlor-anilin,
2,3,4-Trichlor-anilin,
2,3,5-Trichlor-anilin,
2,4,5-Trichlor-anilin,
2,3,6-Trichlor-anilin,
3,4,5-Trichlor-anilin,
2,3,4,6-Tetrachlor-anilin,
2,3,4,5-Tetrachlor-anilin,
2,3,5,6-Tetrachlor-anilin,
Pentachlor-anilin,
3-Chlor-4-brom-5-jod-anilin,
4,5,6-Trichlor-2-methyl-anilin,
2,5-Dichlor-4-methyl-anilin,
2,3,5,6-Tetrachlor-4-methyl-anilin,
2,5-Dichlor-3,4-dimethyl-anilin,
2,5-Dichlor-4-ethyl-anilin,
2,5-Dichlor-4-propyl-anilin,
3,4,6-Trichlor-2-benzyl-anilin,
2,2'-Diamino-3,5,6,3',5',6'-Hexachlor-diphenyl-methan,
3,4,5-Trichlor-2-amino-diphenyl,
4,4'-Diamino-octachlor-diphenyl,
3,4-Dichlor-2-methoxy-anilin,
3,6-Dichlor-2-methoxy-anilin,
4,5-Dichlor-2-methoxy-anilin,
5,6-Dichlor-2-methoxy-anilin,
3,4,6-Trichlor-2-methoxy-anilin,
3,4,5-Trichlor-2-methoxy-anilin,
3,4,5,6-Tetrachlor-2-methoxy-anilin,
4,5-Dichlor-3-methoxy-anilin,
5,6-Dichlor-3-methoxy-anilin,
2,5-Dichlor-3-methoxy-anilin,
4,5,6-Trichlor-3-methoxy-anilin,
2,4,5,6-Tetrachlor-3-methoxy-anilin,
2,3-Dichlor-4-methoxy-anilin,
2,5-Dichlor-4-methoxy-anilin,
2,3,6-Trichlor-4-methoxy-anilin,
2,3,5-Trichlor-4-methoxy-anilin,
2,3,5,6-Tetrachlor-4-methoxy-anilin,
4,5-Dichlor-2-phenoxy-anilin,
3,4,5,6-Tetrachlor-2-phenoxy-anilin,
2,4,5,6-Tetrachlor-3-phenoxy-anilin,
2,5-Dichlor-4-phenoxy-anilin,
2,3,5,6-Tetrachlor-4-phenoxy-anilin.

Das erfindungsgemäße Verfahren wird in Gegenwart von Jodwasserstoff und/oder einer unter den Reaktionsbedingungen des erfindungsgemäßen Verfahrens Jodwasserstoff liefernden Verbindung und bei gleichzeitiger Gegenwart von gegebenenfalls substituierten Phenolen an einem Katalysator aus

Edelmetallen in elementarer oder gebundener Form und gegebenenfalls in Gegenwart eines inerten organischen Lösungs- und/oder Verdünnungsmittels und/oder Wasser durchgeführt.

Der Jodwasserstoff kann in das erfindungsgemäße Verfahren gasförmig oder in Form einer ca. 0,1 bis 65 gew.-%igen bevorzugt einer 10 bis 60 gew.-%igen, wäßrigen Lösung eingesetzt werden. Dabei kann der Jodwasserstoff aus elementarem Jod oder aus Jodverbindungen, die unter den Reaktionsbedingungen des erfindungsgemäßen Verfahrens Jodwasserstoff liefern, in situ hergestellt werden.

Als Jodwasserstoff liefernde anorganische oder organische Verbindungen kommen z. B. Jod, Natriumjodid, Kaliumjodid, $KJ_3$-Lösung, Chlorjod, Jodsäure sowie deren Salze, Jodalkane, wie Jodmethan, Jodethan, 1- und 2-Jodpropan, aromatische Jodverbindungen, wie 2-Jodphenol und 4-Jodphenol, bevorzugt Jod, Natriumjodid, Kaliumjodid, 2-Jodpropan, 2- und 4-Jodphenol sowie Chlorjod in Frage.

Der Jodwasserstoff wird nach dem erfindungsgemäßen Verfahren im allgemeinen in Mengen von 0,01 bis 20 Gew.-%, bevorzugt 0,1 bis 10 Gew.-%, bezogen auf die Gewichtsmenge des zu enthalogenierenden Anilins, eingesetzt. Wird Jod oder eine Jodwasserstoff liefernde Verbindung in das erfindungsgemäße Verfahren eingesetzt, so werden diese üblicherweise in Mengen von 0,01 bis 20 Gew.-%, bevorzugt von 0,1 bis 10 Gew.-%, bezogen auf die Gewichtsmenge des zu enthalogenierenden Anilins, eingesetzt.

Die gegebenenfalls substituierten Phenole werden vorzugsweise unverdünnt in das erfindungsgemäße Verfahren eingesetzt. Es ist jedoch auch möglich, die Phenole in Lösung oder Verdünnung in einem unter den Reaktionsbedingungen inerten organischen Lösungs- und/oder Verdünnungsmittel oder in Wasser einzusetzen.

Als gegebenenfalls substituierte Phenole kommen solche in Frage, die ein- oder mehrfach durch Alkylgruppen mit bis zu 4 Kohlenstoffatomen oder durch Halogen, bevorzugt durch Jod, substituiert sind.

Zum Beispiel werden genannt: Phenol, o-, m-, p-Kresol, 2,3-, 2,4-, 2,5-, 2,6-, 3,5-Xylenol, 2- und 4-Jodphenol. Die gegebenenfalls substituierten Phenole können sowohl einzeln als auch im Gemisch untereinander in das erfindungsgemäße Verfahren eingesetzt werden, z. B. m-/p-Kresol-Gemische.

Die Menge der in das erfindungsgemäße Verfahren einzusetzenden, gegebenenfalls substituierten Phenole kann in weiten Bereichen variiert werden. Üblicherweise werden 1 bis 90 Gew.-%, bevorzugt 30 bis 70 Gew.-%, gegebenenfalls substituierte Phenole, bezogen auf die Menge des Reaktionsgemisches, in das erfindungsgemäße Verfahren eingesetzt.

Als Edelmetalle seien die Elemente der 8. Gruppe des Periodensystems der Elemente (Mendelejew), wie Ruthenium, Rhodium, Palladium, Osmium, Iridium und Platin, bevorzugt Ruthenium, Platin und Palladium, genannt.

Als Edelmetalle in gebundener Form können beispielsweise die Oxide, Sulfide und/oder Polysulfide eingesetzt werden.

Die Katalysatoren nach dem erfindungsgemäßen Verfahren können auch auf Trägermaterialien angewendet werden. Dafür kommen alle an sich bekannten Trägermaterialien in Frage, soweit sie gegen Wasser und Säuren inert sind. Als solche seien Bariumsulfat und Aktivkohle, bevorzugt Aktivkohle, genannt.

Die Herstellung des Edelmetallkatalysators auf einem Trägermaterial kann in an sich bekannter Weise erfolgen. Beispielsweise wird das Trägermaterial in der wäßrigen Lösung des Edelmetalls aufgeschlämmt, und dann das Edelmetall durch Zugabe eines Reduktionsmittels, wie Wasserstoff oder Hydrazin, auf das Trägermaterial gefällt.

Besonders bei der kontinuierlichen Durchführung des erfindungsgemäßen Verfahrens ist es vorteilhaft, den Edelmetallkatalysator auf einem Trägermaterial im Reaktionsraum als Festbett- oder Fließbett-Katalysator anzuordnen.

Die Katalysatoren, die zur Durchführung des erfindungsgemäßen Verfahrens eingesetzt werden, erhalten auch bei mehrmalig wiederholter Verwendung oder bei kontinuierlicher Durchführung des erfindungsgemäßen Verfahrens über lange Zeit ihre Aktivität und ihre Selektivität und ergeben gleichbleibend hohe Ausbeuten.

Die Menge des Katalysators, der zur Durchführung des erfindungsgemäßen Verfahrens eingesetzt wird, beträgt im allgemeinen 0,1 bis 2 Gew.-%, bevorzugt 1 bis 1,5 Gew.-%, bezogen auf das als Ausgangsmaterial verwendete Anilin. Bei Verwendung eines auf einen Träger aufgebrachten Katalysators werden üblicherweise 1 bis 20 Gew.-%, bevorzugt 10 bis 15 Gew.-%, bezogen auf das Ausgangsmaterial, eingesetzt.

Das erfindungsgemäße Verfahren wird gegebenenfalls in Gegenwart von inerten organischen Lösungs- und/oder Verdünnungsmitteln und/oder in Gegenwart von Wasser durchgeführt. Als inerte, organische Lösungs- und/oder Verdünnungsmittel werden beispielsweise Methylalkohol, Ethylalkohol, Benzol, Chlorbenzol, o-Dichlorbenzol, Toluol und Xylol, vorzugsweise Toluol, genannt.

Im allgemeinen wird das erfindungsgemäße Verfahren in saurem Medium durchgeführt. Das saure Medium kann entweder durch den bei der Reaktion entstehenden Halogenwasserstoff oder durch den eingesetzten Jodwasserstoff erzeugt werden.

Bei dem Arbeiten in Gegenwart von Wasser wird das erfindungsgemäße Verfahren im allgemeinen bei einem pH-Wert von kleiner als 4, bevorzugt kleiner als 1, durchgeführt.

Zur Einstellung des sauren Mediums auf einen pH-Wert von $< 1$ kann man dem Gemisch vor Beginn der Reaktion eine kleine Menge Halogenwasserstoff zusetzen. Die Menge des zugesetzten Halogenwasserstoffs wird vorzugsweise sehr gering gehalten, um einem erhöhten Zwangsanfall an Halogenwasserstoff bzw. dessen Salze bei der Aufarbeitung zu vermeiden. Bevorzugt setzt man Jod in phenolischer Lösung, das unter den Reaktionsbedingungen Jodwasserstoff bildet, oder Jodwasserstoff selbst oder eine der bereits genannten Jodverbindungen, die ebenfalls unter den Reaktionsbedingungen Jodwasserstoff liefern, und so die Einstellung eines sauren Mediums bewirken, ein.

Das erfindungsgemäße Verfahren kann anhand der folgenden Reaktionsgleichung für die Entchlorierung von Pentachloranilin zu 3,5-Dichlor-anilin erläutert werden :

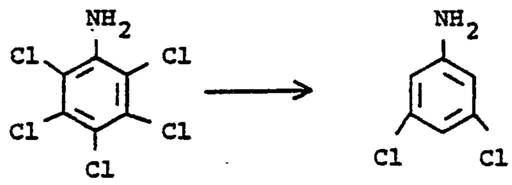

Nach dem erfindungsgemäßen Verfahren ist es möglich, nicht nur von z. B. reinen Tetrachloranilinen oder Pentachloranilin auszugehen, sondern auch von Gemischen aus Tetrachloranilinen und Pentachloranilin, wie sie bei der technischen Herstellung aus Tetrachlorbenzol durch Nitrieren und katalytische Reduktion der Nitrogruppe anfallen.

Weiterhin ist es möglich, als Ausgangsmaterial anstelle der chlorierten Aniline die entsprechenden Nitrochlorverbindungen einzusetzen und in einer Eintopfreaktion die Reduktion der Nitrogruppe und die hydrierende Enthalogenierung durchzuführen.

Im allgemeinen wird das erfindungsgemäße Verfahren so durchgeführt, daß man das Ausgangsmaterial, das Phenol, gegebenenfalls ein Lösungs- oder Verdünnungsmittel, Katalysator und Jodwasserstoff oder eine unter den Reaktionsbedingungen des Verfahrens Jodwasserstoff liefernde Verbindung, in einem säurefesten Autoklav, z. B. einem Autoklav aus Emaille oder Tantal, vorlegt und nach Verschließen des Autoklavs die Luft mit Stickstoff und anschließend den Stickstoff mit Wasserstoff verdrängt.

Zur Durchführung der Reaktion wird der Wasserstoff gasförmig in das Reaktionsgemisch geleitet. Im allgemeinen arbeitet man bei einem Wasserstoffdruck von 10 bis 100 bar, bevorzugt bei 15 bis 60 bar, besonders bevorzugt bei 20 bis 50 bar.

Das erfindungsgemäße Verfahren wird im allgemeinen bei einer Temperatur von 100 bis 250 °C, bevorzugt bei 120 bis 200 °C, besonders bevorzugt bei 140 bis 190 °C, durchgeführt.

Die Reaktionsdauer hängt u. a. von der angewandten Reaktionstemperatur und dem angewandten Wasserstoffdruck ab und liegt bei 170 °C bei etwa 2 Stunden.

Nach beendeter Reaktion kann das Gemisch in an sich bekannter Weise aufgearbeitet werden. Zum Beispiel kann beim Einsatz von Wasser als Lösungs- und/oder Verdünnungsmittel der Katalysator durch Absaugen des heißen Gemisches abgetrennt werden. Die m-halogensubstituierten Aniline können anschließend durch Zugabe von z. B. Alkalihydroxidlösung freigesetzt und mit einem mit Wasser nicht mischbaren Lösungsmittel, z. B. Methylenchlorid oder Toluol, extrahiert werden. Dabei bleibt das Phenol als Alkaliphenolat in Lösung. Aus dem Lösungsmittel können die Halogenaniline beispielsweise durch Destillation erhalten werden.

Bei dem Einsatz eines nicht mit Wasser mischbaren Lösungs- und/oder Verdünnungsmittels können die 3-Halogen- bzw. die 3,5-Dihalogen-aniline durch Zugabe von wäßrigem Alkalihydroxid freigesetzt werden. Anschließend kann das organische Lösungsmittel abgetrennt und daraus das m-halogensubstituierte Anilin, z. B. durch Destillation, isoliert werden.

Eine besondere Form der Aufarbeitung kann darin bestehen, daß man z. B. nach Verdünnen des Reaktionsgemisches mit Wasser, Absaugen des Katalysators und Neutralisation mit z. B. Natronlauge die organische Phase, gegebenenfalls mit Hilfe eines organischen Lösungsmittels, wie Toluol, abtrennt und durch Destillation das jeweilige halogensubstituierte Anilin gewinnt.

Die Wiedergewinnung des Jods kann nach bekannten Methoden erfolgen, wie durch Oxidation des Jodids mit Chlor und Extraktion des dabei entstehenden Jods mit einem geeigneten organischen Lösungsmittel.

In einer besonderen Ausführungsform des erfindungsgemäßen Verfahrens kann man zur Wiedergewinnung des Jods die wäßrige, Jodid enthaltende Phase z. B. mit Wasserstoffperoxid in Gegenwart von wenig Säure, bevorzugt in Gegenwart von Salzsäure, oxidieren, das dabei entstehende Jod in Phenol aufnehmen und diese Jodlösung in einem weiteren Reaktionsansatz wieder verwenden.

Das erfindungsgemäße Verfahren kann sowohl diskontinuierlich als auch kontinuierlich durchgeführt werden.

Das erfindungsgemäße Verfahren hat den Vorteil, daß ohne zusätzliche Zugabe von Salzen, wie Alkali- oder Erdalkalichloriden, oder von Säuren, wie Salzsäure, oder von Schwermetallsalzen, wie sie z. B. in der europäischen Patentanmeldung 0 015 220 genannt werden, unter milden Reaktionsbedingungen bei hohen Konzentrationen an Einsatzmaterial eine selektive Entchlorierung mit hoher Ausbeute durchgeführt werden kann.

Ein weiterer Vorteil des erfindungsgemäßen Verfahrens ist, daß als Ausgangsmaterial auch schwer trennbare Chlor-Anilin-Gemische verwendet werden können, die neben den Polychloranilinen, die in m-Stellung zur Aminogruppe durch Chlor substituiert sind, weitere Chlor- oder Polychloraniline enthalten, bei denen kein Chlor in m-Stellung zur Aminogruppe steht. Diese Verbindungen werden nach dem erfindungsgemäßen Verfahren zu Anilin entchloriert, das sich leicht destillativ abtrennen läßt. Dagegen ist die Auftrennung eines Polychloranilin-Gemisches umständlich und langwierig.

Ferner kann nach dem erfindungsgemäßen Verfahren ein Gemisch aus Tetrachloranilinen und Pentachloranilin, wie es aus einem technisch hergestellten Tetrachlorbenzol-Gemisch durch Nitrieren und katalytische Reduktion der Nitrogruppe zugänglich ist, selektiv und mit hoher Ausbeute zu 3,5-Dichloranilin enthalogeniert werden.

Außerdem ist es möglich, als Ausgangsmaterial anstelle der chlorierten Aniline die entsprechenden Nitrochlorverbindungen einzusetzen und in einer Eintopfreaktion die Reduktion der Nitrogruppe und die hydrierende Enthalogenierung durchzuführen.

Es muß weiterhin als vorteilhaft angesehen werden, daß das bei der Enthalogenierungsreaktion eingesetzte Phenol unter den genannten Reaktionsbedingungen und in Gegenwart von Edelmetallkatalysatoren mit Wasserstoff nicht zu Cyclohexanol oder Cyclohexanon durch Kernhydrierung umgesetzt wird. Dabei kann das Phenol jeweils durch eine einfache Trennoperation, wie Extraktion oder Destillation, wiedergewonnen und wiederverwendet werden. Außerdem stellt das Phenol ein ausgezeichnetes Medium für die Rückführung des bei der erfindungsgemäßen Umsetzung wiedergewonnenen Jods dar, wodurch sich das erfindungsgemäße Verfahren noch wirtschaftlicher gestalten laßt. Der Einsatz von gegebenenfalls substituierten Phenolen bei dem erfindungsgemäßen Verfahren bietet noch einen weiteren wesentlichen Vorteil, da die zu enthalogenierende Verbindung in sehr hoher Konzentration bei gleichzeitig sehr geringer Konzentration an Edelmetallkatalysator eingesetzt werden kann. Dadurch werden besonders hohe, bislang nicht erreichte Raum/Zeit-Ausbeuten erzielt.

Als durch Halogen meta-substituierte Aniline der Formel (II), die nach dem erfindungsgemäßen Verfahren hergestellt werden können, seien beispielsweise genannt :

3-Chlor-anilin,
3,5-Dichlor-anilin,
3-Chlor-5-jod-anilin,
5-Chlor-2-methyl-anilin,
5-Chlor-3-methyl-anilin,
3-Chlor-4-methyl-anilin,
3,5-Dichlor-4-methyl-anilin,
5-Chlor-3,4-dimethyl-anilin,
3-Chlor-4-ethyl-anilin,
3-Chlor-2-benzyl-anilin,
4,4'-Diamino-2,6,2',6'-tetrachlor-diphenyl,
3-Chlor-2-methoxy-anilin,
5-Chlor-2-methoxy-anilin,
3,5-Dichlor-2-methoxy-anilin,
3-Chlor-4-methoxy-anilin,
5-Chlor-3-methoxy-anilin,
3,5-Dichlor-4-methoxy-anilin,
3-Chlor-2-phenoxy-anilin,
3,5-Dichlor-2-phenoxy-anilin,
3,5-Dichlor-4-phenoxy-anilin.

Die nach dem erfindungsgemäßen Verfahren erhältlichen 3-Chlor- bzw. 3,5-Dichlor-aniline sind bekannte Zwischenprodukte und können zur Herstellung von Pflanzenschutzmitteln verwendet werden (DE-PS 1 034 912, DE-OS 2 021 327, DE-OS 1 812 206, DE-OS 1 958 183, US-PS 2 906 614, US-PS 2 655 445, US-PS 3 652 737).

Die nachfolgenden Beispiele sollen das erfindungsgemäße Verfahren verdeutlichen.

## Beispiel 1

In einem Tantal-Autoklaven (0,85 l) werden 200 Teile Phenol, 197 Teile 2,3,5,6-Tetrachloranilin, 20 Teile Platin-Kohle-Katalysator (3,7 %ig) und 15 Teile wäßrige Jodwasserstoffsäure, 57 %ig, mit Wasserstoff bei einem Druck von max. 50 bar und bei 155 °C innerhalb 3,5 Stunden unter Rühren enthalogeniert. Nach Abkühlen des Reaktionsbehälters und nach Entspannen wird das Reaktionsgemisch mit 500 Vol.-Teilen Wasser verdünnt, mit konzentrierter Natronlauge alkalisch gestellt, mit Toluol verrührt und durch Absaugen vom Platin-Kohle-Kontakt getrennt. Die durch Schichttrennen gewonnene organische Phase wird destillativ vom Lösungsmittel befreit. Durch Destillieren über eine einfache Destillationsbrücke erhält man 99,3 %iges 3,5-Dichloranilin in einer Ausbeute von 97 % bei vollständigem 2,3,5,6-Tetrachloranilin-Umsatz.

Beispiel 2

200 Teile Phenol, 131 Teile 2,3,5,6-Tetrachloranilin, 10 Teile wäßrige Jodwasserstoffsäure 57 %ig und 20 Teile Ruthenium-Kohle-Katalysator (5 %ig) werden in einem Tantal-Autoklaven innerhalb 4 Stunden bei einer Temperatur von 165 °C unter Rühren und mit Wasserstoff bei einem Druck von max. 50 bar enthalogeniert. Nach üblicher Aufarbeitung erhält man 90,5 Teile 3,5-Dichloranilin, 99,1 %ig, Ausbeute : 98 % der Theorie.

Beispiel 3

In einem Tantal-Autoklaven (0,85 l) werden 200 Teile Phenol, 131 Teile eines Gemisches von 2,3,4,5- und 2,3,5,6-Tetrachloranilin sowie Pentachloranilin im Gew.-%-Verhältnis von etwa 38 : 60 : 2 mit 20 Teilen Ruthenium-Kohle-Kontakt (5 %ig) und 10 Teilen wäßrige Jodwasserstoffsäure (57 %ig) vermischt und mit Wasserstoff unter einem Druck von max. 50 bar bei einer Temperatur von 165 °C innerhalb 10 Stunden enthalogeniert. Nach Aufarbeitung gemäß der im Beispiel 1 beschriebenen Arbeitsweise erhält man 91,5 Teile 3,5-Dichloranilin, 98,1 %ig, mit einem Gehalt von 0,6 % 3-Chlor-anilin, das destillativ abtrennbar ist. Ausbeute : 98 % der Theorie.

Beispiel 4

Bei Durchführung des Beispiels 2 mit 5 Teilen Pd-Kohle-Kontakt 5 %ig anstelle des Ruthenium-Kohle-Kontaktes erhält man nach 3-stündiger Reaktionsdauer bei 165 °C und nach üblicher Aufarbeitung 88,5 Teile 99 %iges 3,5-Dichloranilin, Ausbeute : 95 % der Theorie.

Beispiel 5

200 Teile Phenol, 131 Teile Tetrachlor-/Pentachlor-Anilin-Gemisch entsprechend der Zusammensetzung wie im Beispiel 3 erwähnt, 1 Teil Ruthenium-Kohle-Katalysator, 5 %ig, der bereits für 10 vorhergehende gleiche Versuche benutzt worden ist, und 12 Teile wäßrige Jodwasserstoffsäure, 57 %ig, werden in einem Tantal-Autoklaven innerhalb 3,5 Stunden bei 165 °C unter Rühren bei einem Wasserstoffdruck von 50 bar umgesetzt. Nach üblicher Aufarbeitung werden 90 Teile 99 %iges 3,5-Dichloranilin isoliert. Ausbeute : 97 % der Theorie.

Beispiel 6

131 Teile Tetrachloranilin-/Pentachloranilin-Gemisch entsprechend der Angaben im Beispiel 3, 200 Teile Phenol, 5,7 Teile Jod sowie 33 Teile eines wasserfeuchten Ruthenium-Kohle-Kontaktes, 5 %ig (23 Teile trocken), werden unter Rühren in einem Tantal-Autoklaven innerhalb 4 Stunden bei 165 °C und unter einem Wasserstoffdruck von max. 50 bar zur Reaktion gebracht. Nach üblicher Aufarbeitung erhält man 87 Teile 3,5-Dichloranilin 98 %ig (93 % Ausbeute).

Beispiel 7

Bei Wiederholung der Reaktion gemäß Beispiel 3, wobei anstelle der wäßrigen Jodwasserstoffsäure 10 Teile 2-Jodpropan eingesetzt werden, erhält man nach Aufarbeitung 89 Teile 3,5-Dichloranilin. Ausbeute : 97 % der Theorie.

Beispiel 8

131 Teile eines Tetrachlor-Pentachloranilin-Gemisches, 200 Teile Phenol und 10 Teile 2-Jodphenol werden in Gegenwart von 12,5 Teilen Ruthenium/Kohle-Katalysator (5 %) in einem Tantal-Autoklaven mit Wasserstoff unter einem Druck von 29 bis 50 bar bei maximal 165 °C innerhalb 8 Stunden unter Rühren umgesetzt. Nach Aufarbeitung des Reaktionsgemisches erhält man 87,5 Teile 3,5-Dichloranilin. Ausbeute : 95 % der Theorie.

Beispiel 9

55,5 Teile 4-Brom-3-chlor-5-jodanilin, 250 Teile Phenol, 8 Teile Jodwasserstoffsäure (57 %ig) und 15 Teile Ruthenium-Kohle-Katalysator (5 %ig) werden mit Wasserstoff bei 23 bis 47 bar Druck und bei 145 °C innerhalb von 2 Stunden unter Rühren umgesetzt. Nach üblicher Aufarbeitung erhält man 38 Teile 3-Chlor-5-jod-anilin. Ausbeute : 90 % der Theorie.

Beispiel 10

163 Teile 2,5-Dichloranilin (99,8 %ig), 250 Teile Phenol, 14 Teile wäßrige Jodwasserstoffsäure, 57 %ig,

und 23 Teile Ruthenium-Kohle-Katalysator, 5 %ig, werden in einem Tantal-Autoklaven 6 Stunden bei 190 °C unter einem Wasserstoffdruck von max. 50 bar gerührt. Nach Aufarbeitung des Reaktionsgemisches analog Beispiel 1 erhält man 119 Teile rohes 3-Chloranilin, das nach GC-Analyse noch 0,2 % Phenol und 3,5 % 2,5-Dichloranilin (Ausgangsmaterial) enthält und durch Destillation gereinigt werden kann.

Beispiel 11

105 Teile 2,3,5,6-Tetrachlor-4-methyl-anilin (99,4 %ig) werden in 250 Teilen Phenol in Gegenwart von 9 Teilen Jodwasserstoffsäure, 57 %ig, und 23 Teilen eines 5 %igen Ruthenium-Kohle-Katalysators 6,5 Stunden bei 165 °C unter 50 bar Wasserstoffdruck in einem Tantal-Autoklaven unter Rühren umgesetzt. Nach Aufarbeitung analog Beispiel 1 erhält man 73,5 Teile 3,5-Dichlor-4-methylanilin, Fp. 55,5 bis 56,5 °C, Kp. 147 bis 150 °C/14 mbar, 98,6 %ig. Die Ausbeute beträgt 96,2 % der Theorie.

Beispiel 12

100 Teile Pentachloranilin, 200 Teile Phenol, 7 Teile Jodwasserstoffsäure, 57 %ig, und 23 Teile Ruthenium-Kohle-Katalysator, 5 %ig, werden 9 Stunden bei 165 °C mit Wasserstoff unter einem Druck von max. 50 bar in einem Tantal-Autoklaven gerührt. Nach der Aufarbeitung analog der im Beispiel 1 beschriebenen Methode erhält man 59 Teile 3,5-Dichloranilin. Die Ausbeute beträgt 92,5 % der Theorie.

Beispiel 13

164 Teile 2,3-Dichloranilin (99,2 %ig), 250 Teile Phenol, 14 Teile Jodwasserstoffsäure, 57 %ig, und 23 Teile Ruthenium-Kohle-Katalysator, 5 %ig, werden 12 Stunden mit Wasserstoff unter einem Druck von 50 bar bei 180 °C gerührt. Nach Aufarbeitung des Reaktionsgemisches entsprechend der im Beispiel 1 beschriebenen Methode erhält man 121 Teile rohes 3-Chloranilin, 96,4 %ig, daneben noch 3,1 % 2,3-Dichloranilin. Durch Destillation erhält man reines 3-Chloranilin, Ausbeute 91,8 % der Theorie.

Beispiel 14

61,5 Teile 2,4,5-Trichloranilin, 200 Teile Phenol, 10 Teile wäßrige Jodwasserstoffsäure, 57 %ig, und 23 Teile Ruthenium-Kohle-Katalysator, 5 %ig, werden in einem Tantal-Autoklaven 4,5 Stunden bei 180 °C mit Wasserstoff unter einem Druck von max. 50 bar gerührt. Nach Aufarbeitung erhält man ein Produkt, das nach GC-Analyse 0,7 % Anilin, 90,1 % 3-Chloranilin und 8,2 % 2,5-Dichloranilin enthält und durch Destillation gereinigt werden kann. Ausbeute : 88 % der Theorie.

Beispiel 15

200 Teile Phenol, 5,7 Teile Jod, 197 Teile Tetrachloranilin-/Pentachloranilin-Gemisch gemäß den Angaben im Beispiel 3, und 20 Teile Ruthenium-Kohle-Katalysator, 5 %ig, werden in einem Tantal-Autoklaven bei 175 °C mit Wasserstoff unter max. 50 bar Druck hydrierend enthalogeniert. Bei der Aufarbeitung löst man das heiße Gemisch in 450 Vol.-Teilen Wasser bei 100 °C, trennt über eine Nutsche den Katalysator ab und neutralisiert das Filtrat mit Natronlauge. Mit Hilfe von Toluol trennt man 3,5-Dichloranilin und Phenol von der Wasserphase ab und destilliert die organische Lösung.

Nach Abdestillieren von Toluol und Wasser wird das Phenol vom 3,5-Dichloranilin destillativ getrennt und kann wiederverwendet werden. Das zurückbleibende rohe 3,5-Dichloranilin wird durch Destillation über eine einfache Destillationsbrücke gereinigt. Man erhält 132 Teile 98 %iges 3,5-Dichloranilin, Ausbeute 94 % der Theorie.

Zur Wiedergewinnung des Jods gibt man 40 Teile Phenol zur Wasserphase, stellt sie mit Salzsäure sauer und oxidiert das Jodid mit wäßriger 35 %iger Wasserstoffperoxidlösung (ca. 4 Teile). Die phenolische Jodlösung wird abgetrennt und bei einem weiteren Reaktionsansatz wiederverwendet. Die Wasserphase ist nach Dampfbehandlung zum Entfernen restlichen Phenols frei von Jod.

Beispiel 16

131 Teile eines Gemisches aus Tetrachloranilin und Pentachloranilin, entsprechend der in Beispiel 3 angegebenen Zusammensetzung, 200 Teile Phenol und 9 Teile Natriumjodid werden in Gegenwart von 22 Teilen eines bereits für 6 gleichartige Reaktionsansätze benutzten, wasserfeuchten Ruthenium-Kohle-Katalysators (5 %ig), entsprechend 15 Teilen trockener Kontakt, mit Wasserstoff bei 165 °C innerhalb von 8 Stunden unter einem Druck von 40 bis max. 50 bar umgesetzt. Nach üblicher Aufarbeitung erhält man bei vollständigem Umsatz des Ausgangsmaterials 91,5 Teile 3,5-Dichloranilin. Ausbeute : 98 % der Theorie.

Beispiel 17

150 Teile eines Gemisches aus Tetrachlor- und Pentachlor-Nitrobenzol, dessen Zusammensetzung

der des Polychloranilin-Gemisches von Beispiel 3 entspricht, 200 Teile Phenol, 10 Teile wäßriger Jodwasserstoffsäure (57 %ig) werden mit Wasserstoff in Gegenwart von 20 Teilen Ruthenium-Kohle-Katalysator (5 %ig), beginnend bei ca. 60 °C bis max. 165 °C bei einem Druck von anfangs 20 bar bis max. 50 bar innerhalb von 10 Stunden umgesetzt. Nach Aufarbeitung erhält man 84,5 Teile 3,5-Dichloranilin. Ausbeute 92 % der Theorie.

Beispiel 18

In einem 0,85 l Tantal-Autoklav werden 46,2 Teile 2,3,4,5-Tetrachloranilin (0,2 Mol), 154 Teile Phenol, 4 Teile Jodwasserstoff (57 %ige wäßrige Lösung) und 3 Teile eines 10 %igen Platin-Kohle-Katalysators vermischt und unter Rühren bei 160 °C mit Wasserstoff unter einem Gesamtdruck von max. 18 bar innerhalb 3 Stunden umgesetzt. Nach üblicher Aufarbeitung erhält man 3,5-Dichloranilin mit einer Ausbeute von 97 % der Theorie bei vollständigem Tetrachloranilin-Umsatz.

Beispiel 19

In einem Tantal-Autoklaven (0,85 l) werden 131 Teile eines Tetrachlor-/Pentachloranilin-Gemisches (siehe Beispiel 3), 300 Teile o-Kresol, 10 Teile wäßriger Jodwasserstoffsäure, 57 %ig, und 10 Teile Ruthenium-Kohle-Katalysator, 5 %ig, vorgelegt und mit Wasserstoff bei 165 °C unter einem Druck von 45-50 bar innerhalb 5 Stunden unter Rühren zur Umsetzung gebracht. Nach Aufarbeitung des Gemisches erhält man 87,5 Teile 3,5-Dichloranilin, 95 % der Theorie.

Vergleichsbeispiel (Zu Beispiel 1 der europäischen Patentanmeldung 0 015 219)

In einem Tantal Autoklaven (0,85 l) werden 131 Teile 2,3,5,6-Tetrachloranilin, 135 Teile 4 normaler Salzsäure, 5,5 Teile Palladium-Kohle-Katalysator, 10 %ig, und 6 Teile 7,6 molarer Kaliumjodid-Lösung (= 7,45 Teile Kaliumjodid) mit Wasserstoff unter einem Druck von 50 bar bei 165 °C 3 Stunden 15 Minuten unter Rühren erhitzt. Nach Aufarbeitung erhält man 120,5 Teile eines Produkt-Gemisches, das zu nur 9,6 % aus 3,5-Dichlor-anilin besteht, 69 % des eingesetzten Tetrachloranilin sind nicht umgesetzt, 20 % bestehen aus Trichloranilin.

Dieses Beispiel belegt die Überlegenheit des erfindungsgemäßen Verfahrens (siehe Beispiel 1) gegenüber der Arbeitsweise nach Beispiel 1 der europäischen Patentanmeldung.

**Ansprüche**

1. Verfahren zur Herstellung von m-halogensubstituierten Anilinen der Formel

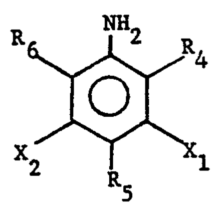

worin

$X_1$ und $X_2$ gleich oder verschieden sind und für Chlor, Brom, Jod, Wasserstoff oder einen gegebenenfalls durch eine Aminogruppe, eine Hydroxygruppe, einen geradkettigen oder verzweigten Alkylrest mit bis zu 12 Kohlenstoffatomen, einen cycloaliphatischen Rest mit 5 bis 6 Kohlenstoffatomen im Ring oder einen Phenylrest substituierten geradkettigen oder verzweigten Alkylrest mit 1 bis 12 Kohlenstoffatomen, cycloaliphatischen Rest mit 5 bis 8 Kohlenstoffatomen im Ring, aromatischen Rest aus der Benzolreihe, Aralkylrest mit 7 bis 18 Kohlenstoffatomen, dessen aliphatischer Teil 1 bis 6 Kohlenstoffatome enthält und dessen aromatischer Teil einen Rest der Benzolreihe darstellt, einen geradkettigen oder verzweigten Alkoxyrest mit 1 bis 12 Kohlenstoffatomen oder einen cycloaliphatischen Alkoxyrest mit 5 bis 6 Kohlenstoffatomen im Ring oder einen Aryloxyrest aus der Benzolreihe stehen, wobei bei der Herstellung von in m-Stellung durch Halogen substituierten Anilinen einer der Reste $X_1$ oder $X_2$ für Chlor, Brom oder Jod steht und bei der Herstellung von 3,5-dihalogensubstituierten Anilinen $X_1$ und $X_2$ für Chlor, Brom oder Jod stehen und

$R_4$, $R_5$ und $R_6$ gleich oder verschieden sind und für Wasserstoff oder für einen, wie oben angegeben, gegebenenfalls substituierten Alkyl-, Aryl-, Aralkyl-, Alkoxy- oder Aryloxyrest stehen

durch Umsetzung von Anilinen der Formel

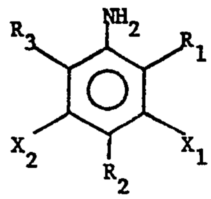

worin

$X_1$ und $X_2$ gleich oder verschieden sind und die obenangegebene Bedeutung haben und $R_1$, $R_2$ und $R_3$ gleich oder verschieden sind und für Chlor, Brom, Jod, Wasserstoff oder einen, wie oben angegeben, gegebenenfalls substituierten Alkyl-, Aryl-, Aralkyl-, Alkoxy- oder Aryloxyrest stehen, wobei wenigstens einer der Reste $R_1$, $R_2$ oder $R_3$ für Chlor, Brom oder Jod steht,

mit Wasserstoff im sauren Medium in Gegenwart von gegebenenfalls auf Trägern aufgebrachten Edelmetallen in elementarer oder gebundener Form und gegebenenfalls in Gegenwart von inerten, organischen Lösungs- und/oder Verdünnungsmitteln und/oder Wasser bei erhöhter Temperatur und erhöhten Drücken, dadurch gekennzeichnet, daß man die Umsetzung in Gegenwart von Jodwasserstoff und/oder von Jodwasserstoff liefernden Verbindungen und bei gleichzeitiger Gegenwart von Phenolen, die ein- oder mehrfach durch Alkylgruppen mit bis zu 4 Kohlenstoffatomen oder durch Halogen substituiert sein können, durchführt.

2. Verfahren nach Anspruch 1, dadurch gekennzeichnet, daß man als Jodwasserstoff liefernde Verbindungen Jod, Natriumjodid, Kaliumjodid, 2-Jodpropan, 2- und 4-Jodphenol und/oder Chlorjod einsetzt.

3. Verfahren nach Ansprüchen 1 und 2, dadurch gekennzeichnet, daß man den Jodwasserstoff und/oder die Jodwasserstoff liefernden Verbindungen in Mengen von 0,01 bis 20 Gew.-%. bezogen auf die Gewichtsmenge des zu enthalogenierenden Anilins, einsetzt.

4. Verfahren nach Ansprüchen 1 bis 3, dadurch gekennzeichnet, daß man als gegebenenfalls substituierte Phenole Phenol, Kresol, Xylenol und/oder Jodphenol einsetzt.

5. Verfahren nach Ansprüchen 1 bis 4, dadurch gekennzeichnet, daß man die gegebenenfalls substituierten Phenole in Mengen von 1 bis 90 Gew.-%, bezogen auf das Gesamtgewicht des Reaktionsgemisches, einsetzt.

6. Verfahren nach Ansprüchen 1 bis 4, dadurch gekennzeichnet, daß man die gegebenenfalls substituierten Phenole in Mengen von 30 bis 70 Gew.-%, bezogen auf das Gesamtgewicht des Reaktionsgemisches, einsetzt.

7. Verfahren nach Ansprüchen 1 bis 6, dadurch gekennzeichnet, daß man die Umsetzung bei Temperaturen von 100 bis 250 °C durchführt.

8. Verfahren nach Ansprüchen 1 bis 7, dadurch gekennzeichnet, daß man die Umsetzung bei einem Druck von 10 bis 100 bar durchführt.

9. Verfahren nach Ansprüchen 1 bis 7, dadurch gekennzeichnet, daß man die Umsetzung bei einem Druck von 20 bis 50 bar durchführt.

10. Verfahren nach Ansprüchen 1 bis 9, dadurch gekennzeichnet, daß man nach Verdünnen des Reaktionsgemisches mit Wasser, Abfiltrieren des Katalysators und Neutralisation die organische Phase abtrennt, die wäßrige Phase in Gegenwart von wenig Säure mit Wasserstoffperoxid oxidiert, das entstehende Jod in Phenol aufnimmt und die phenolische Lösung bei einem weiteren Reaktionsansatz wieder einsetzt.

**Claims**

1. Process for the preparation of m-halogen-substituted anilines of the formula

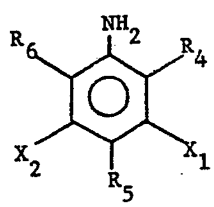

wherein

$X_1$ and $X_2$ are identical or different and represent chlorine, bromine, iodine, hydrogen or a straightchain or branched alkyl radical with 1 to 12 carbon atoms, a cycloaliphatic radical with 5 to 8 carbon atoms in the ring, an aromatic radical from the benzene series, an aralkyl radical with 7 to 18 carbon atoms, the aliphatic part of which contains 1 to 6 carbon atoms and the aromatic part of which represents a radical of the benzene series, a straight-chain or branched alkoxy radical with 1 to 12 carbon atoms or a cycloaliphatic alkoxy radical with 5 to 6 carbon atoms in the ring or an aryloxy radical from the

benzene series, which radicals are optionally substituted by an amino group, a hydroxyl group, a straight-chain or branched alkyl radical with up to 12 carbon atoms, a cycloaliphatic radical with 5 to 6 carbon atoms in the ring or a phenyl radical, one of the radicals $X_1$ or $X_2$ representing chlorine, bromine or iodine in the preparation of anilines substituted in the m-position by halogen and $X_1$ and $X_2$ representing chlorine, bromine or iodine in the preparation of 3,5-dihalogenosubstituted anilines and

$R_4$, $R_5$ and $R_6$ are identical or different and represent hydrogen or an alkyl, aryl, aralkyl, alkoxy or aryloxy radical optionally substituted as indicated above,

by reacting anilines of the formula

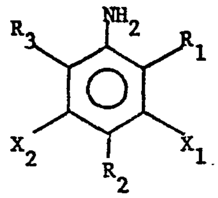

wherein

$X_1$ and $X_2$ are identical or different and have the above-mentioned meaning and

$R_1$, $R_2$ and $R_3$ are identical or different and represent chlorine, bromine, iodine, hydrogen or an alkyl, aryl, aralkyl, alkoxy or aryloxy radical optionally substituted as indicated above, at least one of the radicals $R_1$, $R_2$ or $R_3$ representing chlorine, bromine or iodine,

with hydrogen in an acid medium in the presence of noble metals which are, if appropriate, applied on carriers and are in elementary or bonded form, and if appropriate in the presence of inert organic solvents and/or diluents and/or water, at elevated temperature and elevated pressures, characterised in that the reaction is carried out in the presence of hydrogen iodide and/or compounds yielding hydrogen iodide, phenols which may be mono- or poly-substituted by alkyl groups with up to 4 carbon atoms or by halogen being simultaneously present.

2. Process according to Claim 1, characterised in that iodine, sodium iodide, potassium iodide, 2-iodopropane, 2-iodophenol, 4-iodophenol and/or iodine chloride are employed as compounds yielding hydrogen iodide.

3. Process according to Claims 1 and 2, characterised in that the hydrogen iodide and/or the compounds yielding hydrogen iodide are employed in amounts from 0.01 to 20 % by weight, relative to the amount by weight of the aniline to the dehalogenated.

4. Process according to Claims 1 to 3, characterised in that phenol, cresol, xylenol and/or iodophenol are employed as optionally substituted phenols.

5. Process according to Claims 1 to 4, characterised in that the optionally substituted phenols are employed in amounts from 1 to 90 % by weight, relative to the total weight of the reaction mixture.

6. Process according to Claims 1 to 4, characterised in that the optionally substituted phenols are employed in amounts from 30 to 70 % by weight, relative to the total weight of the reaction mixture.

7. Process according to Claims 1 to 6, characterised in that the reaction is carried out at temperatures of from 100 to 250 °C.

8. Process according to Claims 1 to 7, characterised in that the reaction is carried out under a pressure of from 10 to 100 bars.

9. Process according to Claims 1 to 7, characterised in that the reaction is carried out under a pressure of from 20 to 50 bars.

10. Process according to Claims 1 to 9, characterised in that, after diluting the reaction mixture with water, filtering phase is separated off, the aqueous phase is oxidised with hydrogen peroxide in the presence of a little acid, the iodine formed is taken up in phenol and the phenolic solution is used again in a further reaction cycle.

**Revendications**

1. Procédé pour la fabrication d'anilines m-halogéno-substituées de formule

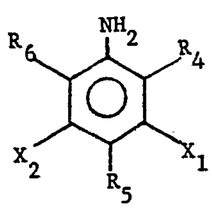

dans laquelle

$X_1$ et $X_2$ sont identiques ou différents et représentent le chlore, le brome, l'iode, l'hydrogène ou un reste alkyle à chaîne droite ou ramifiée en $C_1$-$C_{12}$, un reste cycloaliphatique à cycle en $C_5$-$C_8$, un reste aromatique de la série benzénique, un reste arylalkyle en $C_7$-$C_{18}$ dont la portion aliphatique est en $C_1$-$C_6$ et dont la portion aromatique est un reste de la série benzénique, un reste alcoxy à chaîne droite ou ramifiée en $C_1$-$C_{12}$ ou un reste alcoxy cycloaliphatique, à noyau en $C_5$-$C_6$ ou un reste aryloxy de la série benzénique, éventuellement substitué par un groupe amino, un groupe hydroxy, un groupe alkyle à chaîne droite ou ramifiée jusqu'en $C_{12}$, un reste cycloaliphatique à noyau en $C_5$-$C_6$ ou un reste phényle, avec la condition supplémentaire que, dans le cas de la fabrication d'anilines halogénées dans une position m, l'un des reste $X_1$ et $X_2$ représente le chlore, le brome ou l'iode et, dans le cas de la fabrication d'anilines 3,5-dihalogénées, $X_1$ et $X_2$ représentent le chlore, le brome ou l'iode et

$R_4$, $R_5$ et $R_6$ sont identiques ou différents et représentent le chlore, le brome, l'iode, l'hydrogène ou un reste alkyle, aryle, arylalkyle, alcoxy ou aryloxy éventuellement substitué,

par réaction d'anilines de formule

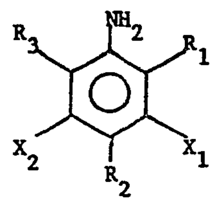

dans laquelle

$X_1$ et $X_2$ sont identiques ou différents et ont la signification indiquée ci-dessus et

$R_1$, $R_2$ et $R_3$ sont identiques ou différents et représentent le chlore, le brome, l'iode, l'hydrogène ou un radical alkyle, aryle, arylalkyle, alcoxy ou aryloxy éventuellement substitué, l'un au moins des restes $R_1$, $R_2$ et $R_3$ étant le chlore, le brome ou l'iode,

avec l'hydrogène en milieu acide, en présence de métaux nobles sous forme élémentaire ou sous forme liée éventuellement déposés sur des supports et éventuellement en présence de solvants et/ou diluants organiques inertes et/ou d'eau à température élevée et sous pressions élevées, caractérisé en ce que l'on met en œuvre la réaction en présence d'iodure d'hydrogène et/ou de composés produisant de l'iodure d'hydrogène et simultanément en présence de phénols facultativement substitués une ou plusieurs fois par des groupes alkyles jusqu'en $C_4$ ou par des halogènes.

2. Procédé selon la revendication 1, caractérisé en ce que l'on utilise comme composé fournissant l'iodure d'hydrogène l'iode, l'iodure de sodium, l'iodure de potassium, le 2-iodopropane, le 2- et le 4-iodophénol et/ou le chlorure d'iode.

3. Procédé selon les revendications 1 et 2, caractérisé en ce que l'on utilise l'iodure d'hydrogène et/ou les composés fournissant l'iodure d'hydrogène en quantités de 0,01 à 20 % en poids, par rapport au poids de l'aniline à déshalogérer.

4. Procédé selon les revendications 1 à 3, caractérisé en ce que l'on utilise comme phénols éventuellement substitués le phénol, le crésol, le xylénol et/ou l'iodophénol.

5. Procédé selon les revendications 1 à 4, caractérisé en ce que l'on utilise les phénols éventuellement substitués en quantités de 1 à 90 % en poids, par rapport au poids total du mélange de réaction.

6. Procédé selon les revendications 1 à 4, caractérisé en ce que l'on utilise les phénols éventuellement substitués en quantités de 30 à 70 % en poids par rapport au poids total du mélange de réaction.

7. Procédé selon les revendications 1 à 6, caractérisé en ce que l'on met en œuvre la réaction à des températures de 100 à 250 °C.

8. Procédé selon les revendications 1 à 7, caractérisé en ce que l'on met en œuvre la réaction sous une pression de 10 à 100 bars.

9. Procédé selon les revendications 1 à 7, caractérisé en ce que l'on met en œuvre la réaction sous une pression de 20 à 50 bars.

10. Procédé selon les revendications 1 à 9, caractérisé en ce que l'on sépare la phase organique après dilution du mélange de réaction par l'eau, séparation du catalyseur par filtration et neutralisation, on oxyde la phase aqueuse par le peroxyde d'hydrogène en présence d'un peu d'acide, on reprend l'iode formé dans le phénol et on réutilise la solution phénolique dans une nouvelle charge de réaction.